# EUROPÄISCHE PATENTSCHRIFT

(11) **EP 3 599 586 B1**
(45) Veröffentlichungstag und Bekanntmachung des Hinweises auf die Patenterteilung: **14.10.2020**
(21) Anmeldenummer: 18185260.9
(22) Anmeldetag: 24.07.2018
(51) Int. Cl.: G06T 7/00

(54) **VERFAHREN UND VORRICHTUNG ZUR ANALYSE VON MAGNETRESONANZ-AUFNAHMEN**
METHOD AND DEVICE FOR ANALYSIS OF MAGNETIC RESONANCE IMAGES
PROCÉDÉ ET DISPOSITIF D'ANALYSE D'IMAGES DE RÉSONANCE MAGNÉTIQUE

(43) Veröffentlichungstag der Anmeldung: 29.01.2020
(73) Patentinhaber: Siemens Healthcare GmbH, 91052 Erlangen (DE)
(72) Erfinder: Forman, Christoph, 91052 Erlangen (DE); Greiser, Andreas, 91054 Erlangen (DE)

(56) Entgegenhaltungen:
- HUELNHAGEN TILL ET AL: "HIGH SPATIAL RESOLUTION MYOCARDIAL T2* MAPPING AT 7.0 T REVEALS DIFFERENCES BETWEEN HEALTHY VOLUNTEERS AND PATIENTS WITH HYPERTROPHIC CARDIOMYOPATHY", INTERNATIONAL SOCIETY FOR MAGNETIC RESONANCE IN MEDICINE, ISMRM, 2030 ADDISON STREET, 7TH FLOOR, BERKELEY, CA 94704 USA, Bd. 23, 15. Mai 2015 (2015-05-15), Seite 2599, XP040668276,
- SALERNO MICHAEL ET AL: "Advances in Parametric Mapping With CMR Imaging", JACC: CARDIOVASCULAR IMAGING, Bd. 6, Nr. 7, 8. Juli 2013 (2013-07-08), Seiten 806-822, XP028672138, ISSN: 1936-878X, DOI: 10.1016/J.JCMG.2013.05.005
- CHRISTOPHER M SANDINO ET AL: "Myocardial T2* mapping: influence of noise on accuracy and precision", JOURNAL OF CARDIOVASCULAR MAGNETIC RESONANCE, BIOMED CENTRAL LTD, LONDON UK, Bd. 17, Nr. 1, 4. Februar 2015 (2015-02-04), Seite 7, XP021213192, ISSN: 1532-429X, DOI: 10.1186/S12968-015-0115-3
- PETER KELLMAN ET AL: "T1 and extracellular volume mapping in the heart: estimation of error maps and the influence of noise on precision", JOURNAL OF CARDIOVASCULAR MAGNETIC RESONANCE, BIOMED CENTRAL LTD, LONDON UK, Bd. 15, Nr. 1, 21. Juni 2013 (2013-06-21), Seite 56, XP021155538, ISSN: 1532-429X, DOI: 10.1186/1532-429X-15-56
- BETTINA BAESSLER ET AL: "Mapping tissue inhomogeneity in acute myocarditis: a novel analytical approach to quantitative myocardial edema imaging by T2-mapping", JOURNAL OF CARDIOVASCULAR MAGNETIC RESONANCE, Bd. 17, Nr. 1, 1. Dezember 2015 (2015-12-01), Seiten 1-11, XP055525806, DOI: 10.1186/s12968-015-0217-y
- SILVA SAMUEL ET AL: "Myocardial Perfusion Analysis from Adenosine-Induced Stress MDCT", 8. Juni 2011 (2011-06-08), INTERNATIONAL CONFERENCE ON SIMULATION, MODELING, AND PROGRAMMING FOR AUTONOMOUS ROBOTS,SIMPAR 2010; [LECTURE NOTES IN COMPUTER SCIENCE; LECT.NOTES COMPUTER], SPRINGER, BERLIN, HEIDELBERG, PAGE(S) 717 - 725, XP047427250, ISBN: 978-3-642-17318-9 * Zusammenfassung * * Abschnitt 5 *

## Beschreibung

Die Erfindung betrifft ein Verfahren und eine Vorrichtung zur Analyse von Magnetresonanz-Aufnahmen, insbesondere zur kombinierten Fehleranalyse und Erzeugung von Inline-Ergebnissen für Quantitatives Magnetresonanz Parameter Mapping.

Bei der Erzeugung von quantitativen Parameterkarten (auch als "Parameter-Maps" bezeichnet), wie z.B. im Rahmen eines T1 Mapping vom Herzen, werden inline Parameter-Maps erzeugt. Um die Maps zu erzeugen werden mehrere Bilder aufgenommen und registriert. Anschließend wird pro Pixel eine Modellfunktion, z.B. im Rahmen eines Inversion Recovery 3-Parameter Fits, angefittet. Die resultierenden Pixel-Maps sollen krankhafte Veränderungen im Herzmuskelgewebe zeigen. Durch manuelles Einzeichnen von Zielregionen (engl.: Regions of Interest; "ROI") können Änderungen in bestimmten Gewebebereichen quantifiziert werden.

Problematisch bei der bisherigen Vorgehensweise ist, dass Artefakte auftreten können, z.B. durch Atembewegungen oder durch variable Herzraten, welche die Ergebnisse verfälschen und schlimmstenfalls als pathologischer Befund interpretiert werden können. Eine Qualitätssicherung ist bisher nicht automatisch gewährleistet.

Bisher wurde dieses Problem weitestgehend dadurch gelöst, dass der Anwender die Qualität der Rohdaten kontrolliert hat sofern er typische Verfälschungen der Maps identifizieren und von echten Läsionen unterscheiden konnte. Alle weiteren Auswertungen mussten offline ausgeführt werden, da auch dort immer eine Interaktion durch einen Anwender notwendig war, um die zu betrachtenden Pixel der ROI (z.B. im Herzgewebe oder im Blut) auszuwählen. 11

Relevanter Stand der Technik ist gegeben durch HUELNHAGEN TILL ET AL: "HIGH SPATIAL RESOLUTION MYOCARDIAL T2* MAPPING AT 7.0 T REVEALS DIFFERENCES BETWEEN HEALTHY VOLUNTEERS AND PATIENTS WITH HYPERTROPHIC CARDIOMYOPATHY", INTERNATIONAL SOCIETY FOR MAGNETIC RESONANCE IN MEDICINE, ISMRM, Bd. 23, 15. Mai 2015, Seite 2599, SALERNO MICHAEL ET AL: "Advances in Parametric Mapping With CMR Imaging", JACC: CARDIOVASCULAR IMAGING, Bd. 6, Nr. 7 , Seiten 806-822, 8. Juli 2013, CHRISTOPHER M SANDINO ET AL: "Myocardial T2* mapping: influence of noise on accuracy and precision", JOURNAL OF CARDIOVASCULAR MAGNETIC RESONANCE, Bd. 17, Nr. 1, 4. Februar 2015, Seite 7, PETER KELLMAN ET AL: "T1 and extracellular volume mapping in the heart: estimation of error maps and the influence of noise on precision", JOURNAL OF CARDIOVASCULAR MAGNETIC RESONANCE, Bd. 15, Nr. 1, 21. Juni 2013, Seite 56, und SILVA SAMUEL ET AL: "Myocardial Perfusion Analysis from Adenosine-Induced Stress MDCT", 8. Juni 2011 , INTERNATIONAL CONFERENCE ON SIMULATION, MODELING, AND PROGRAMMING FOR AUTONOMOUS ROBOTS,SIMPAR 2010, Seiten 717 - 725.

Nachteil des Standes der Technik ist, dass sich das Ergebnis der Untersuchung in der Praxis von dem auswertenden Anwender abhängt, insbesondere von seiner manuellen Auswahl der Pixel.

Es ist eine Aufgabe der vorliegenden Erfindung, ein alternatives, komfortableres Verfahren und eine entsprechende Vorrichtung anzugeben, mit dem die oben beschriebenen Nachteile vermieden werden und insbesondere eine Automatisierung erfolgt, damit stets eine vergleichbare Qualität erreicht werden kann.

Diese Aufgabe wird durch ein Verfahren gemäß Patentanspruch 1, eine Analyseeinheit gemäß Patentanspruch 11, eine Vorrichtung gemäß Patentanspruch 12 und ein medizintechnisches System nach Anspruch 13 gelöst.

Die Erfindung betrifft ein Verfahren und eine Vorrichtung zur Analyse von Magnetresonanz-Aufnahmen. Dies umfasst insbesondere eine kombinierte Fehleranalyse und Erzeugung von Inline-Ergebnissen für quantitatives Magnetresonanz-Parameter Mapping (MR-Parameter Mapping). Eine zentrale Idee der vorliegenden Erfindung ist es insbesondere, die gemessenen Mapping-Daten durch eine systematische, automatisierte (inline) Auswertung bezüglich ihrer Qualität zu bewerten und gleichzeitig - bei ausreichender Qualität - bereits quantitative Ergebnisse gemäß einer standardisierten Auswertung zu erzeugen.

Das erfindungsgemäße Verfahren zur Analyse von Magnetresonanz-Aufnahmen (auch kurz als "MR-Aufnahmen" bezeichnet) umfasst die folgenden Schritte:

### - Bereitstellung einer Bildserie.

Diese Bildserie umfasst eine Anzahl von Magnetresonanz-Aufnahmen, also Aufnahmen, die mittels eines bildgebenden Magnetresonanztomographiesystems angefertigt worden sind. Diese Magnetresonanz-Aufnahmen betreffen zumindest eine Schicht eines Objekts, wobei dieses Objekt in der Regel ein Teil eines menschlichen oder tierischen Patienten ist, z.B. ein Organ. Dabei weisen die Magnetresonanz-Aufnahmen der Bildserie eine Variation eines kontrastbestimmenden Aufnahme-Parameters auf, z.B. im Falle des T1-Mappings eine Variation der Inversionszeit. Es wurden also Bilder der Bilderserie mit einem stets wechselnden Wert für einen kontrastbestimmenden Aufnahme-Parameter aufgenommen. Beispielsweise umfasst die Bildserie mehrere Magnetresonanz-Aufnahmen (z.B. T1-Kontraste) eines menschlichen Herzens, wobei stets dieselbe Schicht durch das Herz mit länger werdenden (oder zumindest unterschiedlichen) Inversionszeiten abgebildet wurde.

Zur Variation des kontrastbestimmenden Aufnahme-Parameters existieren im Stand der Technik Protokolle. Beispielsweise kann ein Wert eines Aufnahme-Parameters innerhalb eines Werteintervalls stetig erhöht oder vermindert werden. Weitere beispielhafte kontrastbestimmende Aufnahme-Parameter sind die Echozeit TE bzw. die T2-Präparationauer, eine Diffusionsgewichtung, eine Flussgewichtung, eine Perfusion oder ein Magnetisierungstransfer.

Das Verfahren kann für Magnetresonanz-Aufnahmen eines einzigen Kontrastes angewandt werden. Es können aber auch mehrere Kontraste aufgenommen werden, und/oder insbesondere mehrere kontrastbestimmende Aufnahme-Parameter variiert werden. Das Verfahren kann dann unabhängig auf die unterschiedlichen Kontraste angewendet werden oder aber auf eine Kombination der Kontraste, wobei eine im Folgenden Verlauf angepasste Modellfunktion (oft auch als "Fitfunktion" bezeichnet) selbstverständlich stets entsprechend der Art der Variation des kontrastbestimmenden Aufnahme-Parameters gewählt werden sollte.

Die Bereitstellung umfasst eine direkte Aufnahme (bzw. eine Messung von quantitativen Mapping-Daten) mittels eines Magnetresonanztomographiesystems oder eine Bereitstellung von vorher angefertigten Magnetresonanz-Aufnahmen aus einem Datenspeicher, z.B. eines PACS (Picture Archiving and Communication System = Bildarchivierungs- und Kommunikationssystem).

Magnetresonanz-Aufnahmen können einzelne (zweidimensionale) Schichten umfassen. Das erfindungsgemäße Verfahren kann aber auch auf 3D Daten angewendet werden. Wichtig ist, dass aus der Bilderserie ein Parameter per Funktions-Fit (siehe folgende Ausführungen) quantitativ ausgewertet werden kann. Es können durchaus mehrere Schichten aufgenommen werden, jedoch ist darauf zu achten, dass im weiteren Verfahrensablauf Bildelemente mit (im Wesentlichen) identischen Raumkoordinaten in den einzelnen Bildern betrachtet werden. Liegen Bilder aus unterschiedlichen Schichten in einer Bilderserie vor, werden im weiteren Verfahrensablauf jeweils diejenigen Bilder betrachtet, die gleiche Raumkoordinaten zeigen.

### - Erzeugung von Karten

Es werden Karten ("Maps") mit Parameterwerten und Fehlern erzeugt. Diese Karten werden zum besseren Verständnis im Folgenden als "Parameter-Maps" und "Error-Maps" bezeichnet, da diese Begriffe international häufig verwendet werden. Auch wenn die Natur der Parameter im Grunde frei gewählt werden kann, werden inline Parameter bevorzugt. Die (ggf. inline) Parameter-Maps und Error-Maps werden durch Fitting von Bildelementen der Magnetresonanz-Aufnahmen der Bilderserie erzeugt. Dabei entsprechen die Bildelemente für einen Fit jeweils identischen Bereichen von identischen Motiven der Bilder der Bildserie bzw. identischen Raumkoordinaten des Objekts. Die Bildelemente umfassen zumeist einzelne Pixel oder Pixelgruppen.

Beispielsweise wird bei einer Bilderserie eines Herzens, bei denen das Herz in seiner Form stets gleich abgebildet wird (z.B. weil die Bilder aufeinander registriert wurden), eine Modellfunktion an stets identische Bereiche der Abbildung des Herzens (z.B. der gleiche Pixel der rechten Herzkammer) angepasst. Diese Anpassung erfolgt über den Wert des jeweiligen Pixels (z.B. Farbe bzw. Helligkeit) in den unterschiedlichen Bildern der Bilderserie, der sich mit der Variation des kontrastbestimmenden Parameters von Bild zu Bild verändert.

Die Modellfunktion umfasst eine mathematische Funktion (z.B. ein Polynom) mit einer Anzahl an Parametern, deren Werte während des Fits (der Anpassung) so bestimmt werden, dass die Funktion die Werte des betreffenden Pixels in allen Bildern der Bildserie annähernd (mit einer möglichst kleinen Abweichung: dem Fehler) beschreiben kann. Die Werte einer Anzahl dieser Parameter (einem einzigen oder mehreren) werden in der Parameter-Map wiedergegeben, die Werte der Fehler werden in der Error-Map wiedergegeben. Beispielsweise wird pro Pixel über die verschiedenen Bilder der Bildserie mit dem Kontrast T2 eine exponentielle Zerfallsfunktion e ^{(-TE/T2)} angefittet, wobei die Pixelwerte jeweils dem gemessenen Kontrast bei der in diesem Beispiel variierten Echozeit TE entsprechen. In diesem Beispiel war TE der kontrastbestimmende Aufnahme-Parameter und in die Parameter-Map werden für die einzelnen Pixel diejenigen Werte für TE eingeordnet, welche die beste Anpassung erlaubten. In die Error-Map werden die Fehlerwerte der jeweiligen Anpassung eingetragen.

Es ist dabei besonders vorteilhaft, wenn als Parameter-Map und Error-Map jeweils ein Bild erstellt wird, bei dem an der Koordinate, an der die jeweiligen untersuchten Bildbereiche (z.B. Pixel) der Bilder der Bildserie lagen, auch die Werte der Parameter eingetragen werden. Wird nun für eine Reihe von Bildbereichen (z.B. Pixel), bevorzugt für jeden Bildbereich, ein solcher Fit ausgeführt, ergibt sich für die Parameter-Map und die Error-Map jeweils ein Bild, welches die Parameterwerte und Fehler(-werte) im Motiv (an der jeweiligen Stelle) wiedergibt. Diese Einordnung hat den besonderen Vorteil, dass die Segmentierung (siehe nachfolgend erläuterten Schritt), deren Skalierung auf den Magnetresonanz-Aufnahmen basiert, sehr einfach auf die Maps abgebildet werden kann.

Weist beispielsweise die Modellfunktion für einzelne Pixel einen einzigen Parameter auf, dann könnte die Parameter-Map des oben angesprochenen Beispiels einer Aufnahme des Herzens das Herz darstellen, wobei jeder Pixel hier den Wert des Parameters wiedergibt, Da sich die Parameterwerte im Bereich des Herzens von der Parameterwerten in der Umgebung des Herzens mit großer Wahrscheinlichkeit unterscheiden, wird in einer Darstellung dieser Parameter-Map das Herz in der Regel deutlich zu erkennen sein.

Die Werte einer Parameter-Map sind also Werte eines oder mehrerer Fitparameter, die Werte einer Error-Map sind die Fehler des entsprechenden Fits. Da es in der Regel für jeden Parameter einen Wert und einen Fehler gibt, kann von einer Parameter-Map und einer "zugehörigen" Error-Map gesprochen werden, wenn die Error-Map den Fehler desjenigen Parameters wiedergibt, dessen Wert in der Parameter-Map steht (gleiches gilt für Parametergruppen). Die Parameter-Map und die Error-Map spannen bevorzugt einen 2D Raum auf, in dem die Messergebnisse pro Pixel bzw. Bildelement dargestellt werden können. Es können alle Werte (der Parameter bzw. Fehler) in einer einzigen jeweiligen Map eingetragen werden, es kann aber auch für jeden einzelnen Parameter (oder eine Gruppe von Parametern) eine individuelle Map erstellt werden.

### - Automatische Segmentierung

Die Magnetresonanz-Aufnahmen werden segmentiert. Dies kann zeitlich unabhängig von dem vorangehenden Fit erfolgen, aber auch zeitlich aufeinanderfolgend. Eine automatische Segmentierung einer medizinischen Bildaufnahme ist dem Fachmann im Grunde bekannt, z.B. eine Segmentierung in AHA-Segmente. Durch die Segmentierung werden die für die nachfolgend beschriebenen Histogramme relevanten Segmente erzeugt.

Es kann nun eine Darstellung der Parameter-Map(s) und der Error-Map(s) der Segmente erfolgen, z.B. in Form einer 2D-Auftragung, bei der eine Parameter-Map neben der zugehörigen Error-Map zusammen mit einer Darstellung der Segmentierung in den einzelnen Maps angezeigt werden.

### - Erzeugung von Histogrammen

Es werden nach Durchführung des Fits und der Segmentierung Histogramme einer Parameter-Map und der zugehörigen Error-Map erzeugt. Dabei werden bevorzugt die Werte der Parameter-Map bzw. die diesem Parameter entsprechenden Fehlerwerte der zugehörigen Error-Map innerhalb eines Segments in jeweils ein Histogramm aufgenommen, wobei die jeweiligen Werte in Bins eingeordnet werden. Zumeist ergeben sich (zumindest bezüglich der Parameter-Map) Histogramme mit einem Häufungswert, der von Segment zu Segment unterschiedlich sein kann. Die automatische Segmentierung macht z.B. Annahmen über die Topologie eines Gewebebereichs, z.B. im genannten Anwendungsbeispiel Annahmen über die ringförmige Struktur der linken Herzkammer im Kurzachsenschnitt.

### - Analyse

Anschließend erfolgt eine Analyse der Histogramme. In dem vorangehenden Beispiel würde eine histogrammbasierte Analyse der AHA-Segmente der Parameter-Maps und Error-Maps erfolgen. Bei der Analyse wird basierend auf dem Histogramm, bevorzugt in Vergleich zu Referenzhistogrammen oder Referenzwerten ermittelt, ob in dem jeweiligen Segment eine Anomalie vorliegt oder nicht. Statistische Analysen der Histogramme sind dabei bevorzugt. Beispielsweise kann eine Verschiebung eines Häufungswertes in einem Histogramm von einem Normalwert auf einen anderen Wert oder die Bildung eines neuen (weiteren) Häufungswertes auf eine krankhafte Veränderung schließen lassen oder auf eine andere Anomalie z.B. einen Anteil vom Blut in Gewebe. Bevorzugte Ausführungen der Analyse werden im Folgenden noch genauer erläutert werden.

### - Ausgabe

Danach folgt eine Ausgabe der Ergebnisse der Analyse. Bevorzugt erfolgt dabei eine Darstellung der Parameter-Maps und der Error-Maps der Segmente zusammen mit den Ergebnissen der Analyse, z.B. in Form einer 2D-Auftragung, bei der eine Parameter-Map neben einer Error-Map zusammen mit einer Darstellung der Segmentierung in den einzelnen Maps ausgegeben werden.

Eine erfindungsgemäße Analyseeinheit zur Analyse von Magnetresonanz-Aufnahmen ist zur Durchführung eines erfindungsgemäßen Verfahrens ausgelegt.

Eine erfindungsgemäße Vorrichtung zur Analyse von Magnetresonanz-Aufnahmen umfasst die folgenden Komponenten:
- Eine Datenschnittstelle zum Empfang einer Bildserie umfassend eine Anzahl von Magnetresonanz-Aufnahmen einer Schicht eines Objekts, wobei die Magnetresonanz-Aufnahmen eine Variation eines kontrastbestimmenden Parameters aufweisen.
- Eine Fitting-Einheit ausgelegt zur Erzeugung von Parameter-Maps und von Error-Maps durch Fitting von Bildelementen der Magnetresonanz-Aufnahmen einer empfangenen Bilderserie.
- Eine Segmentierungs-Einheit ausgelegt zur automatische Segmentierung der Magnetresonanz-Aufnahmen.
- Eine Histogramm-Einheit ausgelegt zur Erzeugung von Histogrammen der Parameter-Maps und von Error-Maps , wobei eine Anzahl von Histogrammen (H) der Parameter-Map (PM) und der Error-Map (EM) gebildet wird, wobei ein Histogramm (H) die Informationen der entsprechenden Parameter-Map (PM) bzw. Error-Map (EM) innerhalb eines Segments (S) umfasst, und eine erfindungsgemäße Analyseeinheit ausgelegt zur Analyse der Histogramme.
- Eine Ausgabeeinheit ausgelegt zur Darstellung der Parameter-Maps und der Error-Maps der Segmente, und/oder zur Ausgabe der Ergebnisse der Analyse.

Ein erfindungsgemäßes medizintechnisches System umfasst eine erfindungsgemäße Vorrichtung zur Analyse von Magnetresonanz-Aufnahmen, wobei das erfindungsgemäße medizintechnische System bevorzugt eine Befundungsstation oder eine Steuereinheit mit dieser Vorrichtung umfasst.

Ein Großteil der zuvor genannten Komponenten der Vorrichtung können ganz oder teilweise in Form von Softwaremodulen in einem Prozessor einer entsprechenden Vorrichtung realisiert werden. Eine weitgehend softwaremäßige Realisierung hat den Vorteil, dass auch schon bisher verwendete Steuereinrichtungen bzw. Befundungsstationen auf einfache Weise durch ein Software-Update nachgerüstet werden können, um auf die erfindungsgemäße Weise zu arbeiten. Insofern wird die Aufgabe auch durch ein entsprechendes Computerprogrammprodukt mit einem Computerprogramm gelöst, welches direkt in eine Vorrichtung ladbar ist, mit Programmabschnitten, um alle Schritte des erfindungsgemäßen Verfahrens auszuführen, wenn das Programm in der Vorrichtung ausgeführt wird. Ein solches Computerprogrammprodukt kann neben dem Computerprogramm gegebenenfalls zusätzliche Bestandteile wie z. B. eine Dokumentation und/oder zusätzliche Komponenten auch Hardware-Komponenten, wie z.B. Hardware-Schlüssel (Dongles etc.) zur Nutzung der Software, umfassen.

Zum Transport zur Vorrichtung und/oder zur Speicherung an oder in dem Rechensystem bzw. der Vorrichtung kann ein computerlesbares Medium, beispielsweise ein Memorystick, eine Festplatte oder ein sonstiger transportabler oder fest eingebauter Datenträger dienen, auf welchem die von einem Rechensystem bzw. einer Rechnereinheit der Vorrichtung einlesbaren und ausführbaren Programmabschnitte des Computerprogramms gespeichert sind. Die Rechnereinheit kann z.B. hierzu einen oder mehrere zusammenarbeitende Mikroprozessoren oder dergleichen aufweisen.

Weitere, besonders vorteilhafte Ausgestaltungen und Weiterbildungen der Erfindung ergeben sich aus den abhängigen Ansprüchen sowie der nachfolgenden Beschreibung.

Gemäß einem bevorzugten Verfahren wird eine Anzahl von Histogrammen einer Parameter-Map und einer Error-Map gebildet. Dabei umfasst ein Histogramm die Informationen der entsprechenden Parameter-Maps und Error-Maps innerhalb eines Segments.

Gemäß einem bevorzugten Verfahren erfolgt die Analyse eines Histogramms für einen Bereich der Parameter-Maps und/oder der Error-Maps mittels Vergleich mit einer Vergleichsgröße, bevorzugt einem Vergleichshistogramm oder einem Vergleichswert. Dabei werden die Ergebnisse aus Unterschieden zwischen Histogramm und Vergleichsgröße ermittelt, in dem die Unterschiede bevorzugt basierend auf vorbekannten Mustern und/oder basierend auf Algorithmen trainiert mit den Prinzipien des maschinellen Lernens (Machine Learning / Deep Learning) analysiert werden.

Bevorzugt werden Vergleichsgrößen verwendet, welche typisch für Anomalien (z.B. für krankes Gewebe) sind oder Vergleichsgrößen, die typisch für den Normalzustand (z.B. für gesundes Gewebe) sind. Es wird diesbezüglich bevorzugt eine Differenz zwischen Histogramm und Vergleichsgröße gebildet und das Ergebnis dieser Differenz weiter untersucht.

Gemäß einem bevorzugten Verfahren erfolgt die Analyse eines Histogramms mit mathematischen Ansätzen für eine Spektralanalyse. Dabei werden bevorzugt Zusatzinformation berücksichtigt. Diese Zusatzinformationen enthalten bevorzugt Angaben zu Patienteneigenschaften (z.B. BMI oder Alter) oder zu Geräteeigenschaften des Gerätes von dem die Magnetresonanz-Aufnahmen stammen. Beispielsweise könnte der Hinweis auf einen hohen BMI eine Analyse bezüglich des Fettgehaltes verbessern.

Die Zusatzinformationen umfassen insbesondere Wertebereiche oder Differenzwerte, bevorzugt bezüglich T1 in Blut, Myokard oder Fett, so dass die relativen Anteile dieser Komponenten identifiziert werden können. Auf Basis der relativen Anteile kann dann auf die Datenqualität (z.B. eine Blutkontamination) oder den klinischen Befund (z.B. eine Fetteinlagerung) geschlossen werden.

Gemäß einem bevorzugten Verfahren erfolgt eine Korrektur der Magnetresonanz-Aufnahmen der Bildserie, insbesondere eine Bewegungskorrektur. Diese Korrektur erfolgt bevorzugt durch eine (insbesondere elastische) Registrierung der Magnetresonanz-Aufnahmen der Bildserie aufeinander. Das weitere Verfahren wird dann mit korrigierten (insbesondere bewegungskorrigierten) Bildern durchgeführt. Die Korrektur ist vorteilhaft, ggf. sogar notwendig, wenn die untersuchten Gewebebereiche Bewegung unterliegen. Das gilt z.B. für das Herz und die Leber, aber auch ggf. für den Kopf. Die Korrektur wird bevorzugt mittels einer Korrektureinheit durchgeführt.

Hier ist es von Vorteil, wenn vor der Korrektur der Magnetresonanz-Aufnahmen der Bildserie die Segmentierung erfolgt. Die Korrektur (also die Registrierung) wird dann vorzugsweise innerhalb der Segmente durchgeführt. Dadurch ergibt sich eine Zeitersparnis und häufig auch eine Steigerung der Qualität der Registrierung. Die Registrierung der Aufnahmen wird also vor dem (insbesondere pixelweisen) Parameterfit durchgeführt.

Gemäß einem bevorzugten Verfahren wird zur Erstellung einer Parameter-Map und/oder einer Error-Map ein bildelementweises Fitting der Parameter durchgeführt. Bevorzugt wird dabei ein Inversion recovery 3-Parameter Fit durchgeführt. Wie bereits vorangehend gesagt wurde, wird bevorzugt pro Pixel eine Modellfunktion angepasst, bevorzugt eine Exponentialfunktion.

Gemäß einem bevorzugten Verfahren erfolgt die automatische Segmentierung der Magnetresonanz-Aufnahmen gemäß eines AHA Segment Modells oder gemäß eines Machine-Learning oder Deep-Learning basierten Verfahrens. Dabei werden Kurzachsen-Schnitte bevorzugt. Das AHA Segment Modell ist dabei besonders gut für das erfindungsgemäße Verfahren geeignet, grundsätzlich kann das Verfahren aber auf alle Gewebebereiche angewendet werden. Die Segmentierung erfolgt bevorzugt mit dem gleichen Kernalgorithmus wie die "InlineVF" oder syngo.via Ventricular Function Analysis.

Gemäß einem bevorzugten Verfahren weisen die Magnetresonanz-Aufnahmen Kontraste der Gruppe T1, insbesondere T1 pre/post, T2, T2*, ECV, Diffusion, Perfusion auf. Bevorzugt findet dabei eine Variation der Inversionszeit (z.B. im Falle des T1 Mappings), eine Variation der Echozeit, der Präparationsdauer, der Diffusionsgewichtung, der Flussgewichtung, der Perfusion oder des Magnetisierungstransfers statt.

Gemäß einem bevorzugten Verfahren erfolgt die Ausgabe der Ergebnisse der Analyse in Form einer zweidimensionalen Auftragung von Parameterwerten der Parameter-Map und Fehlerwerten der Error-Map. Dabei werden insbesondere die Parameter-Map und die Error-Map nebeneinander zusammen mit einer Sichtbarmachung der Segmente, die sich bei der Segmentierung ergeben haben, und zusammen mit den Ergebnissen der Analyse ausgegeben. Mit einer möglichst schnellen Ausgabe können die Daten schon bald nach der Messung hinreichend gut evaluiert werden um z.B. über die Notwendigkeit eines Rescans, ggf. mit angepassten Messparametern, zu entscheiden.

Gemäß einem bevorzugten Verfahren erfolgt im Rahmen der Analyse in einem Segment eine Überprüfung auf vorgegebene Abweichungsmuster und eine Ausgabe einer Empfehlung für eine weitere Messung bei Vorliegen des Abweichungsmusters. Bevorzugt erfolgt eine oder mehrere der folgenden Überprüfungen.
- Überprüfung, ob homogen höhere Werte in der Error-Map vorliegen. Daraus kann dann auf ein SNR Problem geschlossen werden. Im Rahmen der Ausgabe wird bevorzugt eine Überprüfung der Spulen vorgeschlagen.
- Überprüfung, ob lokal höhere Werte nach einer anatomischen Struktur vorliegen. Daraus kann dann auf eine Bewegung geschlossen werden. Im Rahmen der Ausgabe wird bevorzugt eine bessere Atemkontrolle vorgeschlagen.
- Überprüfung, ob lokal erhöhte Werte unabhängig von einer anatomischen Struktur vorliegen. Daraus kann dann auf ein Banding geschlossen werden. Im Rahmen der Ausgabe wird bevorzugt ein patientenspezifischer Shim vorgeschlagen.
- Überprüfung, ob eine starke Variabilität in der Parameter-Map (insbesondere bei T1-Kontrast), aber niedrige Werte in der Error-Map vorliegen. Daraus kann dann auf eine Fetteinlagerung geschlossen werden. Im Rahmen der Ausgabe wird bevorzugt Dixon vorgeschlagen. Wenn z.B. ein großer Fettgehalt ermittelt wird, wird eine zusätzliche Messung auf Fett vorgeschlagen.

Aufgrund der Werteverteilungen der Pixel in einem Segment können Fehlerursachen differenziert und spezifisch adressiert werden.

Das Verfahren ist prinzipiell anwendbar auf alle Mappingverfahren bzw. für alle MR-Aufnahmen, die eine automatische Gewebesegmentierung erlauben und einen Parameter Fit bzgl. einer gemessenen Kenngröße wie z.B. T1, T2, T2*, Diffusion, Perfusion, etc. beinhalten.

Die segmentbasierte Auswertung liefert (sofern die Datenqualität ausreichend ist) bereits zuverlässig Aufschluss über krankhafte Veränderungen. Das Erkennen der Fehlermuster kann durch den Einsatz von Machine Learning bzw. Deep Learning besonders effizient umgesetzt werden.

Aufgrund der automatischen Segmentierung können Ergebnisse von verschiedenen relevanten Parametern (z.B. T1, T2) in einer gemeinsamen Inline Auswertung, ggf. auf koregistrierten Daten, zusammengeführt und so der diagnostische Wert der Messung weiter erhöht werden.

Im Vergleich zum Stand der Technik ermöglicht die vorgeschlagene Einbeziehung von Error Maps, automatischer Inline Segmentierung und einer strukturierten Auswertung eine schnellere Bewertung der Messergebnisse. Damit kann zum einen schon während der Untersuchung reagiert werden, indem die Messung wiederholt oder auf Basis der quantitativen Ergebnisse zusätzliche Messungen initiiert werden, z.B. eine Fat-Water-Separation Messung falls sehr niedrige T1 Werte gemessen werden, um Fetteinlagerungen zu detektieren oder ein Rescan nach Advanced Shim Justage falls Off-Resonanz Artefakte sichtbar sind in den Error Maps.

Damit wird die Häufigkeit von nicht-diagnostischen Messungen verringert und die Diagnosesicherheit erhöht. Als weitere Konsequenz der Inline Auswertung können zeitaufwändige offline Auswertungen vermieden oder zumindest verkürzt werden.

Durch die Aufbereitung der Pixel Map Ergebnisse in Form von durch die Error-Maps als zuverlässig eingestuften Ergebnisse als Histogramme pro Segment/Schicht liegen die Daten bereits in einem geeigneten Format vor, um komplexere statistische Verfahren wie Mustererkennung anzuwenden oder die Histogramm Ergebnisse zusammen mit der Normalwert Verteilung in einen Lernalgorithmus zu füttern, der dann z.B. die typischen Werteverteilungen für bestimmte Krankheitsbilder erkennt.

Die standardisierte Inline Segmentierung und Auswertung ermöglicht es weiterhin, auch die Messergebnisse zu verschiedenen Parametern wie z.B. T1 pre/post, ECV, T2, Perfusion, etc. in der Auswertung bereits inline zusammenzuführen und z.B. in multiparametrischen Cluster Analysen zur Klassifizierung von Gewebeveränderungen heranzuziehen.

Die Erfindung wird im Folgenden unter Hinweis auf die beigefügten Figuren anhand von Ausführungsbeispielen noch einmal näher erläutert. Dabei sind in den verschiedenen Figuren gleiche Komponenten mit identischen Bezugsziffern versehen. Die Figuren sind in der Regel nicht maßstäblich. Es zeigen:
Figur 1 einen Ablaufplan für einen möglichen Ablauf eines erfindungsgemäßen Verfahrens,
Figur 2 eine schematische Darstellung eines Magnetresonanztomographiesystems gemäß einem Ausführungsbeispiel der Erfindung,
Figur 3 eine bevorzugte Darstellung eines möglichen Ergebnisses des erfindungsgemäßen Verfahrens.
Figur 4 eine bevorzugte Darstellung eines weiteren möglichen Ergebnisses des erfindungsgemäßen Verfahrens.
Figur 5 eine bevorzugte Darstellung eines weiteren möglichen Ergebnisses des erfindungsgemäßen Verfahrens.

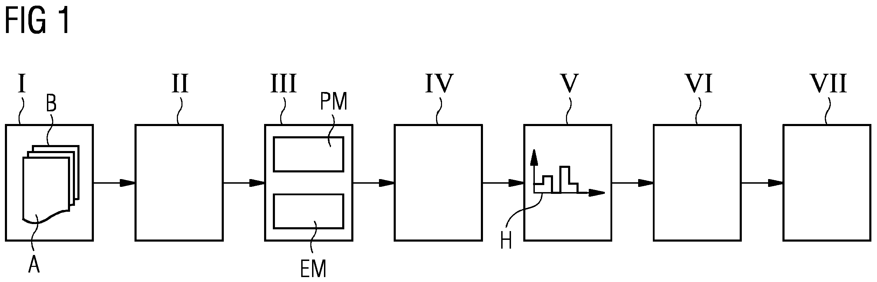

Figur 1 zeigt einen Ablaufplan für einen möglichen Ablauf eines erfindungsgemäßen Verfahrens zur Analyse von Magnetresonanz-Aufnahmen A.

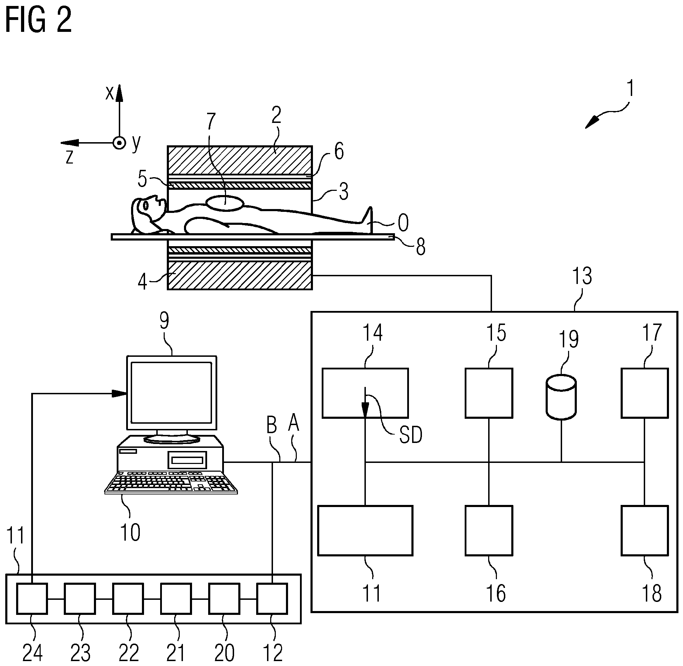

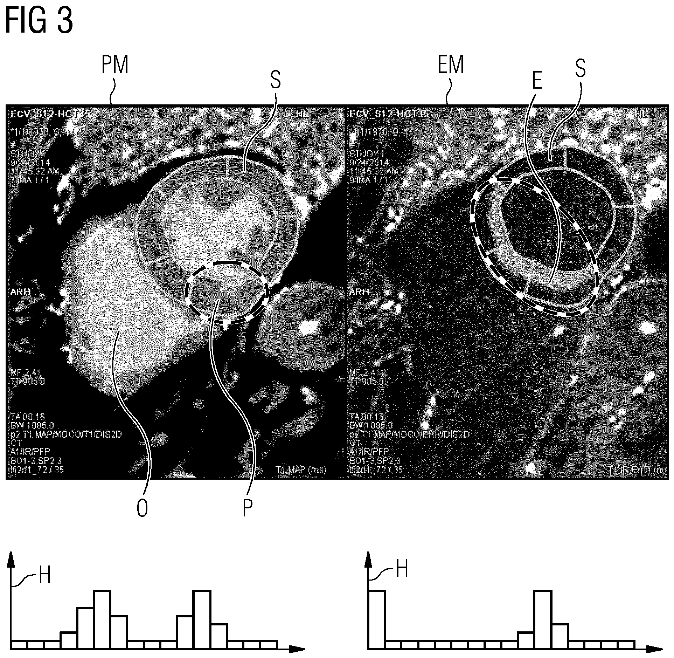

In Schritt I erfolgt eine Bereitstellung einer Bildserie B umfassend eine Anzahl von Magnetresonanz-Aufnahmen A einer Schicht eines Objekts, beispielsweise ein Herz (s. dazu z.B. Figuren 2 bis 5), wobei die Magnetresonanz-Aufnahmen A eine Variation eines kontrastbestimmenden Aufnahme-Parameters aufweisen. Dieser kontrastbestimmenden Aufnahme-Parameter kann z.B. bei Magnetresonanz-Aufnahmen A wie sie für die Figuren 3 bis 5 als Basis dienten und einen T1 Kontrast darstellten, die Inversionszeit sein, die innerhalb eines Zeitintervalls aufsteigend (zu längeren Inversionszeiten hin) oder absteigend (zu kürzeren Inversionszeiten hin) variiert wird.

In Schritt II erfolgt eine Korrektur der Magnetresonanz-Aufnahmen A der Bildserie B, da z.B. ein Herz während der Aufnahme stetig schlägt. Hier kann beispielsweise eine Registrierung der Magnetresonanz-Aufnahmen A der Bildserie B aufeinander erfolgen, wobei eine der Magnetresonanz-Aufnahmen A als Referenzbild festgelegt wird und das Objekt O (z.B. das Herz) in allen anderen relevanten Magnetresonanz-Aufnahmen A auf das Referenzbild registriert werden. Das weitere Verfahren wird dann mit den korrigierten Magnetresonanz-Aufnahmen A durchgeführt. Dieser Schritt kann theoretisch wegfallen, sofern mit bereits idealen Aufnahmen gearbeitet wird.

In Schritt III erfolgt eine Erzeugung einer Parameter-Map PM und einer Error-Map EM. Dabei wird eine Modellfunktion z.B. an die Werte einzelner Pixel der Bilder angepasst, wobei die Pixel jeweils an denselben Bildkoordinaten liegen (und damit jeweils gleiche Bereiche des Objekts zeigen, welches aber mit stetig variierenden kontrastbestimmenden Aufnahme-Parametern aufgenommen worden sind). Die Modellfunktion umfasst mindestens eine Parameter, dessen Wert so lange verändert wird, bis die Modellfunktion gut die Werte der Pixel wiedergibt. Der letztendlich ausgesuchte Parameterwert wird in die Parameter-Map PM eingeordnet (vorzugsweise an die entsprechende Bildkoordinate einer 2D-Auftragung) und der Fehlerwert der Modellfunktion in eine Error-Map EM (vorzugsweise ebenfalls an die entsprechende Bildkoordinate einer 2D-Auftragung).

In Schritt IV erfolgt eine automatische Segmentierung der Magnetresonanz-Aufnahmen A. Ist das Objekt ein Herz, kann die Segmentierung des linken Ventrikels beispielsweise aussehen wie in den Figuren 3 bis 5 dargestellt ist.

In Schritt V erfolgt eine Erzeugung von Histogrammen H der Parameter-Map PM und der Error-Map EM. Dabei werden hier die Parameterwerte P der Parameter-Map PM innerhalb eines Segments in ein Histogramm eingetragen und die Fehlerwerte E der Error-Map EM in ein anderes Histogramm (s. z.B. Figuren 3 bis 5) .

In Schritt VI erfolgt eine Analyse der Histogramme (s. z.B. Beschreibung zu den Figuren 3 bis 5).

In Schritt VII erfolgt eine Ausgabe der Ergebnisse der Analyse zusammen mit einer Darstellung der Parameter-Map PM und der Error-Map EM und der Segmente S.

In Figur 2 ist grob schematisch ein Magnetresonanztomographiesystem 1 dargestellt. Es umfasst zum einen den eigentlichen Magnetresonanzscanner 2 mit einem Untersuchungsraum 3 bzw. Patiententunnel, in den auf einer Liege 8 ein Patient oder Proband positioniert ist, in dessen Körper sich das eigentliche Untersuchungsobjekt O (z.B. das Herz oder die Leber) befindet.

Der Magnetresonanzscanner 2 ist in üblicher Weise mit einem Grundfeldmagnetsystem 4, einem Gradientensystem 6 sowie einem HF-Sendeantennensystem 5 und einem HF-Empfangsantennen-system 7 ausgestattet. In dem dargestellten Ausführungsbeispiel handelt es sich bei dem HF-Sendeantennensystem 5 um eine im Magnetresonanzscanner 2 fest eingebaute Ganzkörperspule. Das Grundfeldmagnetsystem 4 ist hier in üblicher Weise so ausgebildet, dass es ein Grundmagnetfeld in Längsrichtung des Patienten erzeugt, d. h. entlang der in z-Richtung verlaufenden Längsachse des Magnetresonanzscanners 2. Das Gradientensystem 6 umfasst in üblicher Weise einzeln ansteuerbare Gradientenspulen, um unabhängig voneinander Gradienten in x-, y- oder z-Richtung schalten zu können. Zudem enthält der Magnetresonanzscanner 2 (nicht dargestellte) Shimspulen, die in üblicher Weise ausgebildet sein können.

Bei dem in Figur 2 dargestellten Magnetresonanztomographie-System handelt es sich um eine Ganzkörperanlage mit einem Patiententunnel, in den ein Patient komplett eingebracht werden kann. Grundsätzlich kann die Erfindung aber auch an anderen Magnetresonanztomographie-Systemen, z. B. mit seitlich offenem, C-förmigen Gehäuse, verwendet werden. Wesentlich ist nur, dass entsprechende Aufnahmen des Untersuchungsobjekts O angefertigt werden können.

Das Magnetresonanztomographie-System 1 weist weiterhin eine zentrale Steuereinrichtung 13 auf, die zur Steuerung des MR-Systems 1 verwendet wird. Diese zentrale Steuereinrichtung 13 umfasst eine Sequenzsteuereinheit 14. Mit dieser wird die Abfolge von Hochfrequenz-Pulsen (HF-Pulsen) und von Gradientenpulsen in Abhängigkeit von einer gewählten Pulssequenz oder einer Abfolge von mehreren Pulssequenzen zur Aufnahme mehrerer Schichten in einem interessierenden Volumenbereich des Untersuchungsobjekts innerhalb einer Messsitzung gesteuert. Eine solche Pulssequenz kann beispielsweise innerhalb eines Mess- oder Steuerprotokolls vorgegeben und parametrisiert sein. Üblicherweise sind verschiedene Steuerprotokolle für unterschiedliche Messungen bzw. Messsitzungen in einem Speicher 19 hinterlegt und können von einem Bediener ausgewählt (und bei Bedarf gegebenenfalls geändert) und dann zur Durchführung der Messung genutzt werden.

Zur Ausgabe der einzelnen HF-Pulse einer Pulssequenz weist die zentrale Steuereinrichtung 13 eine Hochfrequenzsendeeinrichtung 15 auf, die die HF-Pulse erzeugt, verstärkt und über eine geeignete Schnittstelle (nicht im Detail dargestellt) in das HF-Sendeantennensystem 5 einspeist. Zur Steuerung der Gradientenspulen des Gradientensystems 6, um entsprechend der vorgegebenen Pulssequenz die Gradientenpulse passend zu schalten, weist die Steuereinrichtung 13 eine Gradientensystemschnittstelle 16 auf. Über diese Gradientensystemschnittstelle 16 könnten die Diffusions-Gradientenpulse und Spoiler-Gradientenpulse appliziert werden. Die Sequenzsteuereinheit 14 kommuniziert in geeigneter Weise, z. B. durch Aussendung von Sequenzsteuerdaten SD, mit der Hochfrequenzsendeeinrichtung 15 und der Gradientensystemschnittstelle 16 zur Ausführung der Pulssequenz.

Wie durch ein Einstrahlen von HF-Pulsen und die Erzeugung von Gradientenfeldern geeignete Rohdaten akquiriert und daraus Magnetresonanztomographie-Bilder ("Magnetresonanz-Aufnahmen" A) rekonstruiert werden können, ist dem Fachmann grundsätzlich bekannt und wird hier nicht näher erläutert. Ebenso sind verschiedenste Messsequenzen, wie z. B. EPI-Messsequenzen oder andere Messsequenzen zur Erzeugung von diffusionsgewichteten Bildern, dem Fachmann vom Grundsatz her bekannt.

Die Steuereinrichtung 13 weist außerdem eine (ebenfalls in geeigneter Weise mit der Sequenzsteuereinheit 14 kommunizierende) Hochfrequenzempfangseinrichtung 17 auf, um innerhalb der durch die Pulssequenz vorgegebenen Auslesefenster koordiniert mittels des HF-Empfangsantennensystems 7 Magnetresonanz-Signale zu empfangen und so die Rohdaten zu akquirieren.

Eine Rekonstruktionseinheit 18 übernimmt hier die akquirierten Rohdaten und rekonstruiert daraus Magnetresonanz-Aufnahmen A. Auch diese Rekonstruktion erfolgt in der Regel auf Basis von Parametern, die in dem jeweiligen Mess- oder Steuerprotokoll P vorgegeben sein können. Diese Bilddaten können dann beispielsweise in einem Speicher 19 hinterlegt werden.

Wie im Detail durch ein Einstrahlen von HF-Pulsen und das Schalten von Gradientenpulsen geeignete Rohdaten akquiriert und daraus Magnetresonanz-Bilder A rekonstruiert werden können, ist dem Fachmann grundsätzlich bekannt und wird daher hier nicht näher erläutert. Das erfindungsgemäße Magnetresonanztomographie-System 1 und insbesondere die Steuereinrichtung 13 können darüber hinaus noch eine Vielzahl von weiteren, hier nicht im Einzelnen dargestellten, aber üblicherweise an derartigen Anlagen vorhandenen Komponenten aufweisen, wie beispielsweise eine Netzwerkschnittstelle, um das gesamte System mit einem Netzwerk zu verbinden und Rohdaten und/oder Bilddaten, aber auch weitere Daten, wie beispielsweise patientenrelevante Daten oder Steuerprotokolle, austauschen zu können.

Eine Bedienung der zentralen Steuereinrichtung 13 kann über ein Terminal 10 mit einer Eingabeeinheit und einer Anzeigeeinheit 9 erfolgen, über das somit auch das gesamte Magnetresonanztomographie-System 1 durch eine Bedienperson bedient werden kann. Auf der Anzeigeeinheit 9 können auch Magnetresonanz-Aufnahmen A angezeigt werden, und mittels der Eingabeeinheit, gegebenenfalls in Kombination mit der Anzeigeeinheit 9, können Messungen geplant und gestartet und insbesondere auch eine Befundung vorgenommen werden.

Die Steuereinrichtung 13 kann eine erfindungsgemäße Vorrichtung 11 aufweisen, diese kann sich aber auch in dem Terminal 10 befinden oder an einer externen Befundungsstation, wobei der Terminal 10 auch als Befundungsstation 10 dienen kann. Die in der Steuereinrichtung 13 angedeutete Vorrichtung 11 kann denselben inneren Aufbau aufweisen, wie die an dem Terminal 10 dargestellte.

Die bevorzugte Vorrichtung 11 zur Analyse von Magnetresonanz-Aufnahmen A umfasst:
- Eine Datenschnittstelle 12 zum Empfang einer Bildserie B umfassend eine Anzahl von Magnetresonanz-Aufnahmen A einer Schicht eines Objekts O. Diese Magnetresonanz-Aufnahmen A sollten dabei eine Variation eines kontrastbestimmenden Aufnahme-Parameters aufweisen. Hier wird die Bilderserie B von der Steuereinheit 13 gesendet und umfasst die rekonstruierten Magnetresonanz-Aufnahmen A.
- Eine Korrektureinheit 20 zur Korrektur der Magnetresonanz-Aufnahmen A. Zu der Funktion der Korrektureinheit 20 und zur Funktion der folgenden Komponenten wird auch auf die Beschreibung zu Figur 1 verwiesen.
- Eine Fitting-Einheit 21 ausgelegt zur Erzeugung einer Parameter-Map PM und einer Error-Map EM durch Fitting von Bildelementen der Magnetresonanz-Aufnahmen A der empfangenen Bilderserie B.
- Eine Segmentierungs-Einheit 22 ausgelegt zur automatischen Segmentierung der Magnetresonanz-Aufnahmen (A).
- Eine Histogramm-Einheit 23 ausgelegt zur Erzeugung von Histogrammen H der Parameter-Map PM und der Error-Map EM.
- Eine Analyseeinheit 24 zur Analyse der Histogramme H.

Als Ausgabeeinheit 9 wird hier die Anzeigeeinheit 9 des Terminals 10 verwendet.

Eine mögliche Ausgabe wird in den Figuren 3 bis 5 dargestellt.

Figur 3 zeigt eine bevorzugte Darstellung eines möglichen Ergebnisses des erfindungsgemäßen Verfahrens. Hier sind links die Parameterwerte einer Parameter-Map PM und rechts die Fehlerwerte einer Error-Map EM dargestellt. Die Parameter-Map PM und die Error-Map EM haben hier dasselbe Bildformat wie die ursprünglichen Magnetresonanz-Aufnahmen A. Es ist hier ein Herz, dargestellt, bei dem das linke Ventrikel hier die Region of Interest darstellt. Dieses linke Ventrikel wurde im Bild segmentiert, so dass sich sechs Segmente S ergaben, die annähernd kreisförmig angeordnet sind (es wurde der Übersichtlichkeit halber nur eines der Segmente S mit einem Referenzzeichen versehen). Im linken Bild fällt ein Bereich von besonderen Parameterwerten P auf, der eine mögliche klinisch relevante Struktur bildet. Unter dem linken Bild ist ein Histogramm H dargestellt, das die Parameterwerte des untersten Segments S wiedergibt. Es sind deutlich zwei Häufungswerte zu erkennen, einmal der Hintergrund (linker Häufungswert) und einmal für die besonderen Parameterwerte P (rechter Häufungswert). Dies könnte auf das Vorliegen eines klinischen Befundes hindeuten. Eine Analyse würde den zweiten Häufungswert erkennen, diesen z.B. mit Referenzwerten vergleichen und dadurch zu dem möglichen klinischen Befund gelangen.

Jedoch zeigen sich in diesem Beispiel bei der Betrachtung des rechten Bildes (Error-Map EM) deutlich besondere Fehlerwerte E im untersten Segment S (und auch in den links dazu benachbarten Segmenten). Betrachtet man das zu dem untersten Segment S zugehörige Histogramm H der Fehlerwerte (rechts unten), dann erkennt man eine Häufungswert recht hoher Fehler. Hier würde von der Analyse der Häufungswert der Fehlerwerte erkannt und in diesem Fall eine Überprüfung des Triggers vorgeschlagen werden.

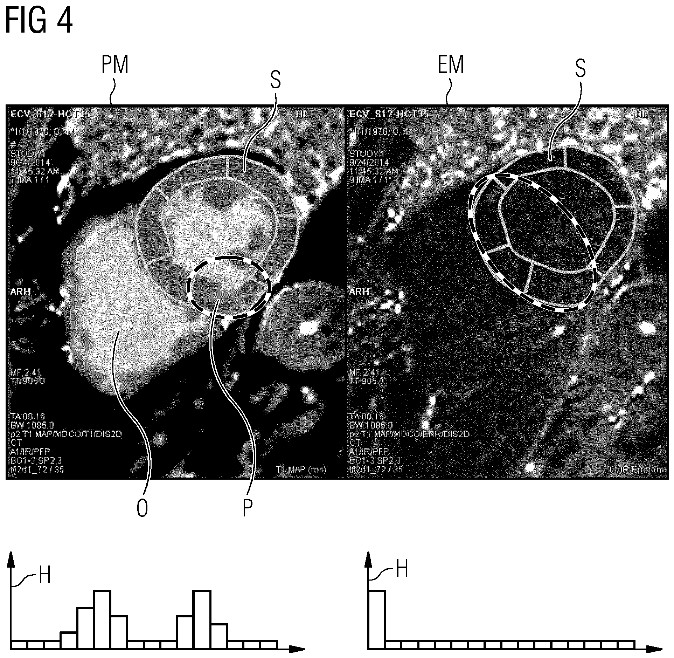

Figur 4 zeigt eine bevorzugte Darstellung eines weiteren möglichen Ergebnisses des erfindungsgemäßen Verfahrens.
Die linke Seite (Parameter-Map PM) ist identisch mit der linken Seite der Figur 3. In der rechten Seite (Error-Map EM) zeigen sich keine besonderen Fehlerwerte. Dies ist auch in dem Histogramm H rechts unten deutlich zu erkennen.

Hier würde seitens der Analyse ein mögliches Vorliegen eines klinischen Befundes erkannt (an dem rechten Häufungswert des Histogramms H links unten) und eine entsprechende Nachricht ausgegeben werden.

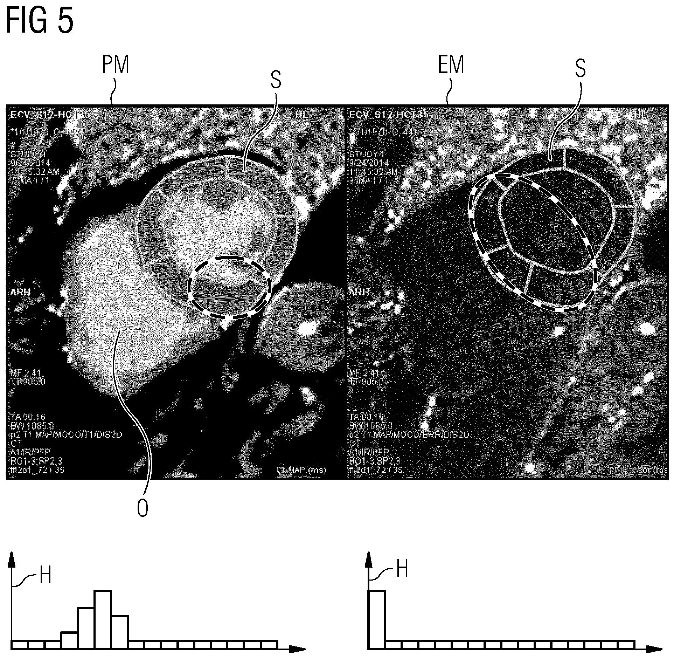

Figur 5 zeigt eine bevorzugte Darstellung eines weiteren möglichen Ergebnisses des erfindungsgemäßen Verfahrens. Hier zeigen weder die linke Seite (Parameter-Map PM) noch die rechte Seite (Error-Map EM) besondere Fehlerwerte oder besondere Parameterwerte P. Demzufolge weisen die Histogramme H links und rechts unten keine besonderen Häufungswerte auf (die eingezeichneten Häufungswerte stehen für die normalen Pixelwerte).

Es wird abschließend noch einmal darauf hingewiesen, dass es sich bei den vorhergehend detailliert beschriebenen Verfahren sowie bei dem dargestellten Magnetresonanztomographiesystem 1 lediglich um Ausführungsbeispiele handelt, welche vom Fachmann in verschiedenster Weise modifiziert werden können, ohne den von den folgenden Ansprüchen definierten Bereich der Erfindung zu verlassen. Weiterhin schließt die Verwendung der unbestimmten Artikel "ein" bzw. "eine" nicht aus, dass die betreffenden Merkmale auch mehrfach vorhanden sein können. Ebenso schließen die Begriffe "Einheit" und "Modul" nicht aus, dass die betreffenden Komponenten aus mehreren zusammenwirkenden Teil-Komponenten bestehen, die gegebenenfalls auch räumlich verteilt sein können.

## Patentansprüche

1. Verfahren zur Analyse von Magnetresonanz-Aufnahmen (A) umfassend die Schritte:
- Bereitstellung einer Bildserie (B) umfassend eine Anzahl von Magnetresonanz-Aufnahmen (A) einer Schicht eines Objekts, wobei die Magnetresonanz-Aufnahmen (A) eine Variation eines kontrastbestimmenden Aufnahme-Parameters aufweisen,
- Erzeugung einer Parameter-Map (PM) und einer Error-Map (EM) durch Fitting von Bildelementen der Magnetresonanz-Aufnahmen (A) der Bilderserie (B),
- Automatische Segmentierung der Magnetresonanz-Aufnahmen (A),
- Erzeugung von Histogrammen (H) der Parameter-Map (PM) und der Error-Map (EM), wobei eine Anzahl von Histogrammen (H) der Parameter-Map (PM) und der Error-Map (EM) gebildet wird, wobei ein Histogramm (H) die Informationen der entsprechenden Parameter-Map (PM) bzw. Error-Map (EM) innerhalb eines Segments (S) umfasst,
- Analyse der Histogramme (H) und/oder
- Darstellung der Parameter-Map (PM) und der Error-Map (EM) der Segmente (S) und/oder Ausgabe der Ergebnisse der Analyse.

2. Verfahren nach Anspruch 1, wobei die Werte der Parameter-Map (PM) bzw. die diesem Parameter entsprechenden Fehlerwerte der zugehörigen Error-Map (EM) innerhalb eines Segments (S) in jeweils ein Histogramm (H) aufgenommen werden, wobei die jeweiligen Werte in Bins eingeordnet werden.

3. Verfahren nach einem der vorangehenden Ansprüche, wobei die Analyse eines Histogramms (H) für einen Bereich der Parameter-Maps (PM) und/oder der Error-Maps (EM) mittels Vergleich mit einer Vergleichsgröße erfolgt, wobei die Ergebnisse aus Unterschieden zwischen Histogramm (H) und Vergleichsgröße ermittelt werden, welche bevorzugt basierend auf vorbekannten Mustern und/oder basierend auf Algorithmen trainiert mit den Prinzipien des maschinellen Lernens analysiert werden.

4. Verfahren nach einem der vorangehenden Ansprüche, wobei die Analyse eines Histogramms (H) mit mathematischen Ansätzen für eine Spektralanalyse erfolgt, wobei bevorzugt Zusatzinformation berücksichtigt werden.

5. Verfahren nach einem der vorangehenden Ansprüche, wobei eine Korrektur der Magnetresonanz-Aufnahmen (A) der Bildserie(B) erfolgt, insbesondere eine Bewegungskorrektur, bevorzugt durch Registrierung der Magnetresonanz-Aufnahmen (A) der Bildserie (B) aufeinander, und das weitere Verfahren mit korrigierten Magnetresonanz-Aufnahmen (A) erfolgt,
wobei bevorzugt vor der Korrektur der Magnetresonanz-Aufnahmen (A) der Bildserie (B) die Segmentierung erfolgt und die Korrektur vorzugsweise innerhalb der Segmente (S) durchgeführt wird.

6. Verfahren nach einem der vorangehenden Ansprüche, wobei zur Erstellung einer Parameter-Map (PM) und/oder Error-Map (EM) ein bildelementweises Anpassung einer Modellfunktion durchgeführt wird, bevorzugt ein Inversion recovery 3-Parameter Fit, wobei bevorzugt pro Pixel eine Modellfunktion angepasst wird, bevorzugt eine Exponentialfunktion.

7. Verfahren nach einem der vorangehenden Ansprüche, wobei die automatische Segmentierung der Magnetresonanz-Aufnahmen (A) gemäß eines AHA Segment Modells oder gemäß eines Machine-Learning oder Deep-Learning basierten Verfahrens erfolgt,

8. Verfahren nach einem der vorangehenden Ansprüche, wobei die Magnetresonanz-Aufnahmen (A) Kontraste der Gruppe T1, insbesondere T1 pre/post, T2, T2*, ECV, Diffusion, Perfusion aufweisen und bevorzugt die Magnetresonanz-Aufnahmen (A) eine Variation der Inversionszeit der Inversionszeit, der Echozeit, der Präparationauer, der Diffusionsgewichtung, der Flussgewichtung, der Perfusion oder des Magnetisierungstransfers aufweisen.

9. Verfahren nach einem der vorangehenden Ansprüche, wobei die Ausgabe der Ergebnisse der Analyse eine zweidimensionale Auftragung von Parameterwerten der Parameter-Map (PM) und Fehlerwerten der Error-Map (EM) umfasst, wobei insbesondere die Parameter-Map (PM) und die Error-Map (EM) nebeneinander zusammen mit einer Sichtbarmachung der Segmente (S) zusammen mit den Ergebnissen der Analyse ausgegeben werden.

10. Verfahren nach einem der vorangehenden Ansprüche, wobei im Rahmen der Analyse in einem Segment (S) eine Überprüfung auf vorgegebene Abweichungsmuster und eine Ausgabe einer Empfehlung für eine weitere Messung bei Vorliegen des Abweichungsmusters erfolgt, bevorzugt eine oder mehrere der folgenden Überprüfungen:
- Überprüfung, ob homogen höhere Werte in der Error-Map (EM) vorliegen und daraus auf ein SNR Problem geschlossen wird und im Rahmen der Ausgabe bevorzugt eine Überprüfung der Spulen vorgeschlagen wird;
- Überprüfung, ob lokal höhere Werte nach einer anatomischen Struktur vorliegen und daraus auf eine Bewegung geschlossen wird und im Rahmen der Ausgabe bevorzugt eine bessere Atemkontrolle vorgeschlagen wird;
- Überprüfung, ob lokal erhöhte Werte unabhängig von einer anatomischen Struktur vorliegen und daraus auf ein Banding geschlossen wird und im Rahmen der Ausgabe bevorzugt ein patientenspezifischer Shim vorgeschlagen wird;
- Überprüfung, ob eine starke Variabilität in der Parameter-Map (PM), aber niedrige Werte in der Error-Map (EM) vorliegen und daraus auf eine Fetteinlagerung geschlossen wird und im Rahmen der Ausgabe bevorzugt Dixon vorgeschlagen wird.

11. Analyseeinheit zur Analyse von Magnetresonanz-Aufnahmen (A), welche zur Durchführung eines Verfahrens nach einem der vorangehenden Ansprüche ausgelegt ist.

12. Vorrichtung (11) zur Analyse von Magnetresonanz-Aufnahmen (A) umfassend:
- eine Datenschnittstelle (12) zum Empfang einer Bildserie (B) umfassend eine Anzahl von Magnetresonanz-Aufnahmen (A) einer Schicht eines Objekts (O), wobei die Magnetresonanz-Aufnahmen (A) eine Variation eines kontrastbestimmenden Aufnahme-Parameters aufweisen,
- Optional: eine Korrektureinheit (20) zur Korrektur der Magnetresonanz-Aufnahmen (A),
- eine Fitting-Einheit (21) ausgelegt zur Erzeugung einer Parameter-Map (PM) und einer Error-Map (EM) durch Fitting von Bildelementen der Magnetresonanz-Aufnahmen (A) einer empfangenen Bilderserie (B),
- eine Segmentierungs-Einheit (22) ausgelegt zur automatischen Segmentierung der Magnetresonanz-Aufnahmen (A),
- eine Histogramm-Einheit (23) ausgelegt zur Erzeugung von Histogrammen (H) der Parameter-Map (PM) und der Error-Map (EM), wobei eine Anzahl von Histogrammen (H) der Parameter-Map (PM) und der Error-Map (EM) gebildet wird, wobei ein Histogramm (H) die Informationen der entsprechenden Parameter-Map (PM) bzw. Error-Map (EM) innerhalb eines Segments (S) umfasst,
- eine Analyseeinheit (24) ausgelegt zur Analyse der Histogramme, und/oder
- eine Ausgabeeinheit (9) ausgelegt zur Darstellung der Parameter-Map (PM) und der Error-Map (EM) der Segmente (S), und/oder zur Ausgabe der Ergebnisse der Analyse.

13. Medizintechnisches System (1), umfassend eine Vorrichtung (11) zur Analyse von Magnetresonanz-Aufnahmen (A) nach Anspruch 12, wobei das erfindungsgemäße medizintechnische System bevorzugt eine Befundungsstation (9) oder eine Steuereinrichtung (13) mit dieser Vorrichtung (11) umfasst.

14. Computerprogrammprodukt mit einem Computerprogramm, welches direkt in eine Speichereinrichtung einer Recheneinrichtung, insbesondere eines medizintechnischen Systems (1), ladbar ist, mit Programmabschnitten, um alle Schritte des Verfahrens nach einem der Ansprüche 1 bis 10 auszuführen, wenn das Computerprogramm in der Recheneinrichtung ausgeführt wird.

15. Computerlesbares Medium, auf welchem von einer Rechnereinheit einlesbare und ausführbare Programmabschnitte gespeichert sind, um alle Schritte des Verfahrens nach einem der Ansprüche 1 bis 10 auszuführen, wenn die Programmabschnitte von der Rechnereinheit ausgeführt werden.

## Claims

1. Method for analysing acquired magnetic resonance images (A), comprising the steps:
- providing an image series (B) comprising a number of acquired magnetic resonance images (A) of a slice of an object, wherein the acquired magnetic resonance images (A) have a variation of a contrast-determining acquisition parameter,
- generating a parameter map (PM) and an error map (EM) by fitting picture elements of the acquired magnetic resonance images (A) of the image series (B),
- automatic segmentation of the acquired magnetic resonance images (A),
- generating histograms (H) of the parameter map (PM) and the error map (EM), wherein a number of histograms (H) of the parameter map (PM) and the error map (EM) are formed, wherein a histogram (H) comprises the information of the corresponding parameter map (PM) and error map (EM) within a segment (S),
- analysing the histograms (H)
and/or
- visualising the parameter map (PM) and the error map (EM) of the segments (S) and/or outputting the results of the analysis.

2. Method according to claim 1, wherein the values of the parameter map (PM) and the error values corresponding to said parameter in the associated error map (EM) within a segment (S) are incorporated in a respective histogram (H), the respective values being sorted into bins.

3. Method according to one of the preceding claims, wherein the analysis of a histogram (H) for a region of the parameter maps (PM) and/or the error maps (EM) is conducted by means of comparison with a comparison variable, wherein the results are determined from differences between histogram (H) and comparison variable which are analysed preferably based on previously known patterns and/or based on algorithms trained using the principles of machine learning.

4. Method according to one of the preceding claims, wherein the analysis of a histogram (H) is carried out using mathematical approaches for a spectral analysis, wherein supplementary information is preferably taken into account.

5. Method according to one of the preceding claims, wherein the acquired magnetic resonance images (A) of the image series (B) undergo a correction, in particular a motion correction, preferably by registration of the acquired magnetic resonance images (A) of the image series (B) on one another, and the further method is carried out using corrected acquired magnetic resonance images (A),
wherein the segmentation is preferably performed prior to the correction of the acquired magnetic resonance images (A) of the image series (B) and the correction is preferably performed within the segments (S).

6. Method according to one of the preceding claims, wherein a model function, preferably an inversion recovery 3-parameter fit, is fitted picture element by picture element in order to generate a parameter map (PM) and/or an error map (EM), wherein a model function, preferably an exponential function, is preferably fitted pixel by pixel.

7. Method according to one of the preceding claims, wherein the automatic segmentation of the acquired magnetic resonance images (A) is performed in accordance with an AHA segment model or according to a machine-learning- or deep-learning-based method.

8. Method according to one of the preceding claims, wherein the acquired magnetic resonance images (A) have contrasts of the T1 group, in particular T1 pre/post, T2, T2*, ECV, diffusion, perfusion, and preferably the acquired magnetic resonance images (A) have a variation of the inversion time, the echo time, the preparation duration, the diffusion weighting, the flow weighting, the perfusion or the magnetisation transfer.

9. Method according to one of the preceding claims, wherein the output of the results of the analysis comprises a two-dimensional plot of parameter values of the parameter map (PM) and error values of the error map (EM), wherein in particular the parameter map (PM) and the error map (EM) are output next to each other together with a visualisation of the segments (S) together with the results of the analysis.

10. Method according to one of the preceding claims, wherein a check is carried out within the scope of the analysis for predefined deviation patterns in a segment (S) and a recommendation for a further measurement is output if the deviation pattern is present, wherein preferably one or more of the following checks are conducted:
- check whether homogeneously higher values are present in the error map (EM) and inferring an SNR problem therefrom, and preferably a check of the coils is suggested within the scope of the output;
- check whether locally higher values according to an anatomical structure are present and inferring a movement therefrom, and preferably a better breath control is suggested within the scope of the output;
- check whether locally increased values are present independently of an anatomical structure and inferring a banding therefrom, and preferably a patient-specific shim is suggested within the scope of the output;
- check whether there is a strong variability in the parameter map (PM), though low values are present in the error map (EM) and inferring a fat accumulation therefrom, and preferably Dixon is suggested within the scope of the output.

11. Analysis unit for analysing acquired magnetic resonance images (A), which is configured for conducting a method according to one of the preceding claims.

12. Device (11) for analysing acquired magnetic resonance images (A) comprising:
- a data interface (12) for receiving an image series (B) comprising a number of acquired magnetic resonance images (A) of a slice of an object (O), wherein the acquired magnetic resonance images (A) have a variation of a contrast-determining acquisition parameter,
- optionally: a correction unit (20) for correcting the acquired magnetic resonance images (A),
- a fitting unit (21) configured for generating a parameter map (PM) and an error map (EM) by fitting picture elements of the magnetic resonance images (A) of a received image series (B),
- a segmentation unit (22) configured for the automatic segmentation of the acquired magnetic resonance images (A),
- a histogram unit (23) configured for generating histograms (H) of the parameter map (PM) and the error map (EM), wherein a number of histograms (H) of the parameter map (PM) and the error map (EM) are formed, wherein a histogram (H) comprises the information of the corresponding parameter map (PM) and error map (EM) within a segment (S),
- an analysis unit (24) configured for analysing the histograms, and/or
- an output unit (9) configured for visualising the parameter map (PM) and the error map (EM) of the segments (S), and/or for outputting the results of the analysis.

13. Medical system (1) comprising a device (11) for analysing acquired magnetic resonance images (A) according to claim 12, wherein the medical system according to the invention preferably comprises a diagnostic assessment station (9) or a control facility (13) including said device (11).

14. Computer program product comprising a computer program which can be loaded directly into a memory facility of a computing facility, in particular a medical system (1), and having program sections for performing all steps of the method according to one of claims 1 to 10 when the computer program is executed in the computing facility.

15. Computer-readable medium on which are stored program sections that can be read in and executed by a computer unit in order to perform all steps of the method according to one of claims 1 to 10 when the program sections are executed by the computer unit.

## Revendications

1. Procédé d'analyse d'enregistrements (A) de résonance magnétique, comprenant les stades :
- on se procure une série (B) d'images comprenant un certain nombre d'enregistrements (A) de résonance magnétique d'une couche d'un objet, les enregistrements (A) de résonance magnétique ayant une variation d'un paramètre d'enregistrement déterminant le contraste,
- on produit une carte (PM) de paramètre et une carte (EM) d'erreur par ajustement d'éléments d'image des enregistrements (A) de résonance magnétique de la série (B) d'images,
- on segmente automatiquement des enregistrements (A) de résonance magnétique,
- on produit des histogrammes (H) de la carte (PM) de paramètre et de la carte (EM) d'erreur, dans lequel on forme un certain nombre d'histogrammes (H) de la carte (PM) de paramètre et de la carte (EM) d'erreur, dans lequel un histogramme (H) comprend les informations de la carte (PM) de paramètre correspondante ou, respectivement, de la carte (EM) d'erreur au sein d'un segment (S),
- on analyse les histogrammes (H)
et /ou
- on représente la carte (PM) de paramètre et la carte (EM) d'erreur des segments (S) et /ou on émet les résultats de l'analyse.

2. Procédé suivant la revendication 1, dans lequel on enregistre les valeurs de la carte (PM) de paramètre ou les valeurs d'erreur, correspondant à ce paramètre, de la carte (EM) d'erreur associée au sein d'un segment (S) dans, respectivement, un histogramme (H), les valeurs respectives étant classées en binaires.

3. Procédé suivant l'une des revendications précédentes, dans lequel l'analyse d'un histogramme (H) pour une région de la carte (PM) de paramètre et /ou de la carte (EM) d'erreur s'effectue au moyen d'une comparaison à une grandeur de comparaison, dans lequel on détermine les résultats à partir de différences entre l'histogramme (H) et la grandeur de comparaison, lesquels, de préférence, sont analysés sur la base de modèles connus à l'avance et /ou sur la base d'algorithmes ayant subi un apprentissage suivant les principes de l'apprentissage automatique.

4. Procédé suivant l'une des revendications précédentes, dans lequel l'analyse d'un histogramme (H) s'effectue par des équations mathématiques pour une analyse spectrale, des informations supplémentaires étant de préférence prises en compte.

5. Procédé suivant l'une des revendications précédentes, dans lequel une correction des enregistrements (A) de résonance magnétique de la série (B) d'images est effectuée, notamment une correction de déplacement, de préférence par enregistrement des enregistrements (A) de résonance magnétique de la série (B) d'images les uns sur les autres et le reste du procédé s'effectue avec des enregistrements de résonance magnétique corrigée,
dans lequel de préférence, avant la correction des enregistrements (A) de résonance magnétique de la série (B) d'images, on effectue la segmentation et on effectue la correction de préférence au sein des segments (S).

6. Procédé suivant l'une des revendications précédentes, dans lequel, pour l'établissement d'une carte (PM) de paramètre et /ou d'une carte (EM) d'erreur, on effectue une adaptation, élément d'image par élément d'image, d'une fonction de modèle, de préférence une inversion recovery 3-parameter fit, dans lequel on adapte, de préférence par pixel, une fonction de modèle, de préférence une fonction exponentielle.

7. Procédé suivant l'une des revendications précédentes, dans lequel on effectue la segmentation automatique des enregistrements (A) de résonance magnétique suivant un AHA segment modell ou suivant un procédé reposant sur un apprentissage automatique ou un apprentissage profond.

8. Procédé suivant l'une des revendications précédentes, dans lequel les enregistrements (A) de résonance magnétique ont des contrastes du groupe T1, notamment T1 pre /post, T2, T2*, ECV, de diffusion, de perfusion et, de préférence, les enregistrements (A) de résonance magnétique ont une variation du temps d'inversion, du temps d'écho, de la durée de préparation, de la pondération de diffusion, de la pondération de flux, de la perfusion ou du transfert d'aimantation.

9. Procédé suivant l'une des revendications précédentes, dans lequel la sortie des résultats de l'analyse comprend un rapport en deux dimensions des valeurs de paramètre de la carte (PM) de paramètre et des valeurs d'erreur de la carte (EM) d'erreur, dans lequel notamment la carte (PM) de paramètres et la carte (EM) d'erreur sont sorties ensemble avec les résultats de l'analyse, les unes à côté des autres, ensemble avec une visualisation des segments (S).

10. Procédé suivant l'une des revendications précédentes, dans lequel, dans le cadre de l'analyse, on effectue dans un segment (S) un contrôle sur un modèle d'écart donné à l'avance et une émission d'une recommandation pour une autre mesure en présence du modèle d'écart, en appliquant de préférence l'un ou plusieurs des contrôles suivant :
- contrôle du point de savoir s'il y a des valeurs assez hautes d'une manière homogène dans la carte (EM) d'erreur et si on en déduit un problème SNR et, dans le cadre de l'émission, on propose de préférence un contrôle des bobines ;
- contrôle s'il y a des valeurs assez hautes localement suivant une structure anatomique et si on en déduit un mouvement et, dans le cadre de l'émission, de préférence on propose un contrôle respiratoire meilleur ;
- contrôle s'il y a des valeurs élevées localement, indépendamment d'une structure anatomique et si on en déduit un banding et, dans le cadre de l'émission, on propose de préférence un shim spécifique au patient ;
- contrôle s'il y a une variabilité forte dans la carte (PM) de paramètre, mais des valeurs plus basses dans la carte (EM) d'erreur et si on en déduit un dépôt de graisse et, dans le cadre de l'émission, on propose de préférence un dixon.

11. Unité d'analyse d'enregistrements (A) de résonance magnétique, qui est conçue pour effectuer un procédé suivant l'une des revendications précédentes.

12. Installation (11) d'analyse d'enregistrements (A) de résonance magnétique, comprenant :
- une interface (12) de données pour recevoir une série (B) d'images comprenant un certain nombres d'enregistrements (A) de résonance magnétique d'une couche d'un objet (O), les enregistrements (A) de résonance magnétique ayant une variation d'un paramètre d'enregistrement déterminant le contraste,
- facultativement : une unité (20) de correction des enregistrements (1) de résonance magnétique,
- une unité (21) d'ajustement, conçue pour produire une carte (PM) de paramètre et une carte (EM) d'erreur par ajustement d'éléments d'image des enregistrements (A) de résonance magnétique d'une série (B) d'images reçue,
- une unité (22) de segmentation, conçue pour segmenter automatiquement les enregistrements (A) de résonance magnétique,
- une unité (23) d'histogramme, conçue pour produire des histogrammes (H) de la carte (PM) de paramètre et de la carte (EM) d'erreur, un certain nombre d'histogrammes (H) de la carte (PM) de paramètre et de la carte (EM) d'erreur étant formés, un histogramme (H) comprenant les informations de la carte (PM) de paramètre ou de la carte (EM) d'erreur correspondante au sein d'un segment (S),
- une unité (24) d'analyse, conçue pour l'analyse des histogrammes et /ou
- une unité (9) d'émission, conçue pour représenter la carte (PM) de paramètre et la carte (EM) d'erreur des segments (S) et /ou pour sortir les résultats de l'analyse.

13. Système (1) de la technique médicale, comprenant une installation (11) d'analyse des enregistrements (A) de résonance magnétique suivant la revendication 12, dans lequel le système de technique médicale suivant l'invention comprend de préférence un poste (9) de constatation ou un dispositif (13) de commande ayant cette installation (11).

14. Produit de programme d'ordinateur comprenant un programme d'ordinateur, qui peut être chargé directement dans un dispositif de mémoire d'un dispositif informatique, notamment d'un système (1) de la technique médicale, comprenant des parties de programme pour effectuer tous les stades du procédé suivant l'une des revendications 1 à 10, lorsque le programme d'ordinateur est réalisé dans le dispositif informatique.

15. Support déchiffrable par ordinateur, sur lequel sont mises en mémoire des parties de programme pouvant être lues et réalisées par une unité informatique pour effectuer tous les stades du procédé suivant l'une des revendications 1 à 10, lorsque les parties de programme sont réalisées par l'unité informatique.
